# EUROPEAN PATENT SPECIFICATION

(11) **EP 2 275 423 B1**
(45) Date of publication and mention of the grant of the patent: **08.02.2012**
(21) Application number: 09009122.4
(22) Date of filing: 13.07.2009
(51) Int. Cl.: C07D 471/04

(54) **Process for the synthesis of paliperidone**
Verfahren zur Synthese von Paliperidon
Procédé de synthèse de palipéridone

(43) Date of publication of application: 19.01.2011
(73) Proprietor: KRKA, 8501 Novo mesto (SI)
(72) Inventor: Ruzic, Milos, 3000 Celje (SI); Pecavar, Anica, 8000 Novo mesto (SI); Prudic, Darja, 8000 Novo mesto (SI); Plaper, Igor, 8220 Smarjeske Toplice (SI); Klobcar, Andrej, 8000 Novo mesto (SI); Hvala, Jernej, 1000 Ljubljana (SI); Plevnik, Miha, 1295 Ivancna Gorica (SI)
(74) Representative: Westendorp | Sommer

(56) References cited:
- WO-A-2009/010988
- WO-A-2009/047499

## Description

### FIELD OF THE INVENTION

The invention describes an improved process for the synthesis and purification of paliperidone.

### BACKGROUND OF THE INVENTION

Paliperidone, 3-[2-[4-(6-fluoro-1,2-benzisoxazol-3-yl)-1-piperidinyl]ethyl]-6,7,8,9-tetrahydro-9-hydroxy-2-methyl-4H-pyrido[1,2-a]pyrimidin-4-one, is a 5-HT antagonist belonging to the chemical class of benzisoxazole derivatives and a racemic mixture has the following structural formula (I):

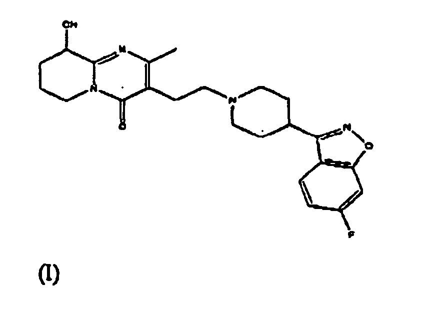

Paliperidone is a primary active metabolite of risperidone, Marketed under the name Invega^{®} paliperidone is a psychotropic agent approved for the treatment of schizophrenia.

A process for the synthesis of paliperidone, is described e.g. in EP 0 368 388 where 6-fluoro-3-(4-pyperidinyl)-1,2-benzisoxazole or its acid addition salts of formula (II) 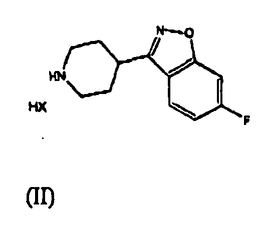 is reacted with 3-(2-chloroethyl)-9-hydroxy-2-methyl-6,7,8,9-tetrahydro-4H-pyrido[1,2-a]pyrimidin-4-one of formula (III):

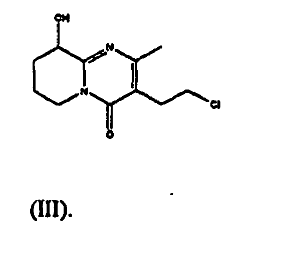

The process is performed in the presence of an organic base and involves isolations using column chromatography and re-crystallizations in suitable solvents, The most persistant impurity to be removed from the active ingredient is 3-[2-[4-(6-fluoro-1,2-benzoisoxazol-3-yl)-1-piperidinyl]ethyl]-2-methyl-7,8-dihydro-4H-pyrido[1,2-a]pyrimidin-4,9(6H)-dione (IV) of formula 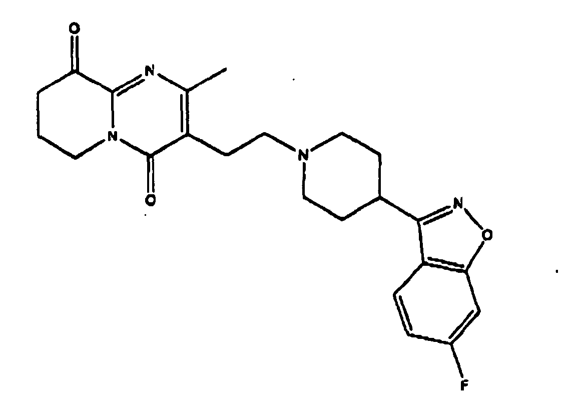 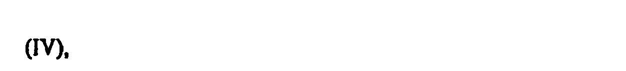 which is very difficult to eliminate due to its chemical similarity with paliperidone, its keto-enol tautomery and its ability to form hydrates,

A process for the synthesis of CMHTP is described in EP 0 368 388, WO 2008/024415, WO 2008/140646, WO 2009/010988, WO 2009/045489 and WO 2009/047499.

WO 2008/021345 describes the synthesis of paliperidone from 3-(2-chloroethyl)-6,7,8,9-tetrahydro-9-hydroxy-4H-pyrido[1,2-a]pyrimidin-4-one and 6-fluoro-3-(4-piperidinyl)-1,2-benzisoxazole in the presence of an inorganic base. The chemical yields are slightly improved with regard to the process disclosed in EP 0 368 388, but on the other hand the organic solvents applied are very volatile and difficult to handle on industrial scale.

There is a need in the art for a new process for preparing paliperidone and its intermediates.

### SUMMARY OF THE INVENTION

The present application relates to an improved process for preparing paliperidone of formula (I) by combining 3-(2-chloroethyl)-6,7,8,9-tetrahydro-9-hydroxy-2-methyl-4H-pyrrido[1,2-a]-pyrimidin-4-one (CMHTP) or a salt thereof and 6-fluoro-3-piperidino-1,2-benzisoxazol (FBIP) or a salt thereof in an organic solvent and in the presence of water, wherein the pH value of the reaction medium is between 6 and 14, preferably between 8 and 11, and wherein active charcoal is used in the crystallization step(s), the active charcoal having a neutral or alkaline pH value when dispersed in water and a content of individual metal cations (e.g. Zn²⁺, Fe²⁺ cations) less than 200 ppm, leading to a product with a much reduced content of the impurities (IV and others) and wherein tedious purification steps may be omitted in comparison with prior processes for the preparation of paliperidone.

### DETAILED DESCRIPTION OF THE INVENTION

The present application relates to an improved process for preparing paliperidone of formula (I) by combining 3-(2-chloroethyl)-6,7,8,9-tetrahydro-9-hydroxy-2-methyl-4H-pyrrido[1,2-a]-pyrimidin-4-one (CMHTP) or a salt thereof and 6-fluoro-3-piperidino-1,2-benzisoxazol (FBIP) or a salt thereof in an organic solvent and in the presence of water, wherein the pH value of the reaction medium is between 6 and 14, preferably between 8 and 11, and wherein active charcoal is used in the crystallization step(s), the active charcoal having a neutral pH value when dispersed in water and a content of individual metal cations (e.g. 2n²⁺, Fe²⁺ cations) less than 200 ppm, leading to a product with a much reduced content of the impurity (IV and other impurities) and wherein tedious purification steps may be omitted in comparison with prior processes for the preparation of paliperidone.

Preferably, the reaction occurs in mixtures of an organic solvent, such as Cl-8 alkyl alcohols, acetonitrile, C3-6 amides, C3-6 ketones, C5-12 aromatic hydrocarbons, C2-6 alkyl acetates and C2 -8 ethers, with water.

Preferred Cl-4 alkyl alcohols are methanol, ethanol, n-propanol, isopropanol (IPA), n-butanol, isobutanol and 2-butanol. Preferred C3-6 amides are dimethylacetamide and dimethylformamide (DMF). Preferred C3-6 ketones are acetone, methyl ethyl ketone (MEK) and methyl iso-butyl ketone (MIBK). Preferred C2-6 alkyl acetates are ethyl acetate and isobutyl acetate. Preferred C2-8 ethers are tetrahydrofurane (THF), diethoxymethane (DEM), isobutyl methyl ether, dibutyl ether and polyethylene glycol (PGME).

Most preferably, the reaction is carried out in a mixture of methanol and water. The water may either be added to the reaction mixture directly, or indirectly, comprised in the base, e.g. diisopropylamine or diisopropylethylamine. In case it is added indirectly, the water content of the base, e.g. diisopropylamine or diisopropylethylamine, has to be determined prior to the reaction, and the missing fraction added directly to the reaction mixture.

In the process of the present invention for preparing paliperidone, the added water fraction in the solvent mixture used can be from 0.5 % (V/V) to 10% (V/V). Preferably the water fraction in the solvent mixture is from 0.5 to 5.0, most preferably from 0.5 to 2.0%.

In the process of the present invention for preparing paliperidone, the inorganic or organic base used can be in a ratio of about 1 to about 3 moles of the base per mole of CMHTP such as about 2.5 moles of the base per mole of CMHTP. The amount of the base used, preferably, is in a molar ratio of about 2, i.e., about 2 moles of the base per mole of CMHTP, such as about 1.8 moles of the base per mole of CMHTP.

Preferably, the reaction occurs under nitrogen (oxygen is degassed from the reaction mixture) and protected from light, in order to avoid the color formation, attributed to some of the impurities.

Typically, the obtained reaction mixture is heated, preferably to a temperature of about 50 °C to about reflux, most preferably to a temperature of about 65 °C to about 70 °C. In a preferred embodiment, the reaction temperature is achieved by slow heating. Preferably such a temperature gradient is applied that a reaction mixture of about 200 to 300 g is heated to the reaction temperature in more than 60 minutes, preferably more than 75 minutes.

The heated mixture is preferably maintained for at least about 5 hours, for the reaction to take place. Preferably, the reaction mixture is maintained for at least about 15 hours, and most preferably, for at least about 30 hours.

Subsequently, the reaction mixture is cooled or the product may be hot filtered. If cooling is applied, it is gradual, to about room temperature, preferably to a temperature of about 30 °C and between 30 and 90 minutes. As used herein, "room temperature" relates to a temperature of about 20 °C to about 25 °C.

After the cooling step, solid paliperidone is formed, which is then recovered by methods known in the art, such as filtration or centrifugation. Preferably, the obtained paliperidone is first washed with an organic solvent, which is the organic solvent used in the reaction, such as methanol, acetonitrile, acetone, dichloromethane or IPA, optionally followed by drying. Preferably, the drying is performed at about 50 °C for about 3 - 6 h.

Both CMHTP and FBIP starting materials can be in the form of a base or acid addition salts. Most preferred salt is a hydrochloride salt. CMHTP may be obtained by any method known in the art, such as the ones described in EP 0 368 388, WO 2008/024415, WO 2008/140646, WO 2009/010988, WO 2009/045489 and WO 2009/047499. FBIP can generally be prepared by any known process such as the processes described in EP 0 196 132, EP 0 428 437, EP 0 081 104, J Med Chem, 1985, vol.28, no.6, p.761.769, Indian J Chem B Org, 2004, vol.43, no.9, p.1954-1957.

CMHTP may contain less than 0.5%, preferably less than 0.2% and more preferably less than 0.05% of impurity of the following formula (V)

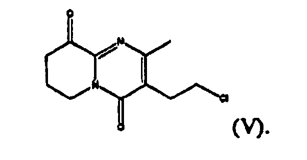

The crude paliperidone according to the present invention, either still wet with a water/methanol content of 3 % w/w to below 1 % w/w directly from the filtration/centrifugation step, or first dried until the loss on drying is bellow 1 % w/w, is macerated in water. The ratio between the mass of the wet or dry crude paliperidone and the volume of water used is between 0.2 g/mL and 0.05 g/mL. The maceration in water is carried out at a temperature between 60 and 99 °C, preferably between 85 and 95 °C. The paliperidone may further be purified, for example, by re-crystalliz,ation such as recrystallization from isopropanol, in the presence of an active charcoal having a neutral pH value when dispersed in water and a content of metal cations (each) less than 200 ppm, as determined by atomic absorption spectrometry.

Optionally, the particle size may be optimized to the average diameter of 20 to 60 micrometers, preferably to the average diameter from 20 - 30 micrometers prior to its isolation with the use of an ultraturrax. In this way, paliperidone with a narrow distribution function is prepared, and the process is easily controlled and repeatable without the need for a second milling process. As the ultraturrax any commercially available product may be used, such as Ultra-Turrax^{®}.

The obtained crystalline form of paliperidone is characterized by powder X-ray diffraction (PXRD) pattern having peaks at about: 8,3, 10.4, 14.7, 15.1, 16.3, 18.8, 20.2, 20.8, 22.2, 24.8, 25.2 and 28.1 ± 0.2 degrees two theta.

The XRD diffractogram was recorded on a Phillips PW3040/60 X'Pert PRO diffractometer using CuKα radiation (1,541874 A).

The content of the impurity (IV) in the product obtained is below 0.15 % as determined with the following HPLC analytical method:

| | |
|---|---|
| **Column:** | Gemini NX C18, 150 x 4,6 mm, 3,0µm |
| **Eluent A:** | 0.02M NaH₂PO₄, pH adjusted to 4.0 with phosphoric acid |
| **Eluent B:** | mixture of acetonitrile and methanol in the ratio 30:70 |

### Gradient:

| t (min) | % A | % B |
|---|---|---|
| 0 | 80 | 20 |
| 5 | 80 | 20 |
| 20 | 65 | 35 |
| 25 | 55 | 45 |
| 30 | 20 | 80 |
| 38 | 20 | 80 |
| 39 | 80 | 20 |

| | |
|---|---|
| **Post time:** | 4 min |
| **Flow-rate:** | 0.7 ml/min |
| **Detection:** | UV, 238 nm |
| **Injection volume:** | 5 µl |
| **Column temperature:** | 40°C |
| **Diluent:** | buffer pH 4.0 and acetonitrile in the ratio 1:1 |

### Sample preparation:

Accurately weigh about 5 mg of sample into 10 mL volumetric flask, dissolve and dilute to volume with diluent.

### Calculation:

Use area per cent method. Do not integrate solvent peaks.

The present invention is illustrated by the following Examples without being limited thereto.

### Examples

### Example 1

### Synthesis of crude paliperidone

20.00 g of 6-fluoro-3-(4-piperidinyl)-1,2-benzisoxazol hydrochloride; 19.82 g of 3-(2-chloroethyl)-9-hydroxy-2-methyl-6,7,8,9-tetrahydro-4h-pyrido[1,2-a]pyrimidin-4-one; from 23.62 g to 27.54 g (33 mL to 38,4 mL) of dry diisopropylamine; 0.70 mL of water and 100 mL to 140 mL of methanol were charged to a 500 mL three-necked flask at room temperature, pH of suspension is 10,1. The reaction was carried out in inert atmosphere (N₂) and protected from light. The suspension was heated to 65 °C to 67 °C in 90 minutes until a clear solution was obtained, After one hour the reaction product began to precipitate from the reaction mixture. The reaction mixture was left at 65 - 67 °C for 30 hours, cooled to 30 °C in 60 minutes, stirred for 30 minutes at 30 °C and filtered. The product was washed with 40 mL of methanol. 27,5 g of paliperidone with a water content of 0,5 % were isolated, Purity was 98.6 area %, level of impurity (IV) was 0.12 area %.

### Example 2

### Maceration of crude paliperidone in water

10.1 g of the wet crude paliperidone obtained as described in Example 1 were suspended in 100 mL of demineralized water under inert atmosphere (N₂) and heated to 97 - 99°C. The suspension was stirred for 15 minutes and hot filtered. The product was washed with 20 mL of water and dried until LOD (Loss on Drying) was below 1% (w/w). 8.84 g of dry paliperidone were obtained, Purity was 98.8 area %, level of impurity (IV) was 0.12 area %.

### Example 3A

### Crystallization of macerated paliperidone

9.20 g of the wet macerated paliperidone of Example 2 (containing 9.08 g of the dry product) were charged to a 1000 mL three-necked flask under inert atmosphere (N₂) and dissolved in 644 mL 2-propanol. 0.46 g of active charcoal (metal cations less than 200 ppm) was added. The mixture was heated to reflux (82 °C) and stirred for 15 minutes. The suspension was hot filtered over randalite and the filtrate was cooled to room temperature. Crystallization of the product was provoked by seeding (cca 2% (w/w). The product started to precipitate between 56 °C to 50 °C. After the filtration, the product was washed with 13.8 mL of 2-propanol. 8.12 g of the wet product were obtained. The product may be dried in a vacuum drier at 50 °C or directly used in a second crystallization. Preferably, the wet product is directly used. Purity was 99.5 area %, level of impurity (IV) was 0.12 area %.

### Example 3B

### Crystallization of macerated paliperidone

9.20 g of the wet macerated paliperidone of Example 2 (containing 9.08 g of the dry product) were charged to a 1000 mL three-necked flask under inert atmosphere (N₂) and dissolved in 644 mL 2-propanol, 0.46 g of active charcoal (metal cations less than 200 ppm) was added. The mixture was heated to reflux (82 °C) and stirred for 15 minutes, The suspension was hot filtered over randalite and the filtrate was cooled to room temperature. Crystallization of the product was provoked with the use of the ultraturrax. The product started to precipitate between 56 °C to 50 °C. After the filtration, the product was washed with 13.8 mL of 2-propanol. 8.12 g of the wet product were obtained. The product may be dried in a vacuum drier at 50 °C or directly used in a second crystallization, Preferably, the wet product is directly used. Purity was 99.5 area %, level of impurity (IV) was 0.12 area %.

The product obtained according to Examples 3A and 3B can be further recrystallized in order to improve the purity of the product.

### Example 4

### Synthesis of crude paliperidone

1.04 kg of 6-fluoro-3-(4-piperidinyl)-1,2-benzisoxazol hydrochloride, 1.03 kg of 3-(2-chloroethyl)-9-hydroxy-2-methyl-6,7,8,9-tetrahydro-4h-pyrido[1,2-a]pyrimidin-4-one; 1.72 L of dry diisopropylamine, 0.04 L of water and 5.2 L of methanol were charged to a reactor at room temperature. The reaction was carried out in inert atmosphere (N₂) and protected from light. The suspension was heated to 65 °C to 67 °C in 120 minutes until a clear solution was obtained. The reaction mixture was left at 65 - 67 °C for 30 hours, cooled to 30 °C in 125 minutes, stirred for 30 minutes at 30 °C and filtered. The product was washed with 2 L of methanol. 1.69 kg of wet paliperidone (1.53 kg of dry crude paliperidone) with a solvent content of 9.6 % were isolated. Purity was 98.2 area %, level of impurity (IV) was 0.12 area %.

### Example 5

### Maceration of crude paliperidone in water

1.69 kg of the wet crude paliperidone obtained as described in Example 4 were suspended in 15.3 L of demineralized water under inert atmosphere (N₂) and heated to 97 - 99 °C. The suspension was stirred for 15 minutes and hot filtered. The product was washed with 1.2 L of water and dried until LOD (Loss on Drying) was below 1% (w/w). 1.4 kg of wet paliperidone were obtained. Purity is 98,5 area %, level of impurity (IV) was 0.12 area %.

### Example 6

### Crystallization of macerated paliperidone

1.4 kg of the dry macerated paliperidone of Example 5 were charged to a reactor under inert atmosphere (N₂) and dissolved in 98 L 2-propanol. 0.06 g of active charcoal (metal cations less than 200 ppm) was added. The mixture was heated to reflux (82 °C) and stirred for 15 minutes. The suspension was hot filtered over randalite and the filtrate was cooled to room temperature. The product started to precipitate between 56 °C to 50 °C. After the filtration, the product was washed with 2 L of 2-propanol. 1.23 kg of the wet product were obtained. The product was dried in a vacuum drier at 50 °C. Purity was 99.5 area %, level of impurity (IV) was 0.12 area % and mass of dry product was 1.13 kg.

### Example 7

### Synthesis of crude paliperidone

1.04 kg of 6-fluoro-3-(4-piperidinyl)-1,2-benzisoxazol hydrochloride, 1.03 kg of 3-(2. chloroethyl)-9-hydroxy-2-methyl-6,7,8,9-tetrahydro-4h-pyrido[1,2-a]pyrimidin-4-one; 1.72 L of dry diisopropylethylamine, 0.04 L of water and 5.2 L of methanol were charged to a reactor at room temperature. The reaction was carried out in inert atmosphere (N₂) and protected from light. The suspension was heated to 65 °C to 67 °C in 140 minutes until a clear solution was obtained. The reaction mixture was left at 65 - 67 °C for 30 hours, cooled to 30 °C in 50 minutes, stirred for 30 minutes at 30 °C and filtered. The product was washed with 2.5 L of methanol. 1.65 kg of wet paliperidone (1.50 kg of dry crude paliperidone) with a solvent content of 9.3 % were isolated. Purity was 98.2 area %, level of impurity (IV) was 0.06 area %.

### Example 8

### Maceration of crude paliperidone in water

1.65 kg of the wet crude paliperidone obtained as described in Example 7 were suspended in 15 L of demineralized water under inert atmosphere (N₂) and heated to 97 - 99 °C. The suspension was stirred for 15 minutes and hot filtered, The product was washed with 1.2 L of water and dried until LOD (Loss on Drying) was below 1% (w/w). 1.5 kg of wet paliperidone were obtained. Purity is 98.6 area %, level of impurity (IV) was 0.06 area %.

### Example 9

### Crystalization of macerated paliperidone

1.4 kg of the dry macerated paliperidone of Example 8 were charged to a reactor under inert atmosphere (N₂) and dissolved in 98 L 2-propanol. 0.06 g of active charcoal (metal cations less than 200 ppm) was added. The mixture was heated to reflux (82 °C) and stirred for 15 minutes. The suspension was hot filtered over randalite and the filtrate was cooled to room temperature. The product started to precipitate between 56 °C to 50 °C. After the filtration, the product was washed with 2 L of 2-propanol. 1.11 kg of the wet product were obtained. The product was dried in a vacuum drier at 50 °C. Purity was 99.4 area %, level of impurity (IV) was 0.06 area % and mass of dry product was 1.11kg.

### Reference experiments which prove the influence of metal cations on purity of substance:

### Example 10

2 g of macerated paliperidone from Example 5 were suspended in 140 mL of 2-propanol and 0.1 g of active charcoal (Zn more than 200 ppm) was added. The mixture was heated to reflux (82 °C) and stirred for 15 minutes. The suspension was hot filtered over randalite and the filtrate was cooled to room temperature. The product started to precipitate between 56 °C to 50 °C. After the filtration, the product was washed with 4 mL of 2-propanol, Purity of wet product was 93.1 area %, level of impurity (IV) was 1.57 area %.

### Example 11

1 g of paliperidone (level of impurity (IV) was 0.75 area %), 30 mL of water and 1.57 g ZnCl₃ (anhydrous) were charged in 50 mL three-necked flask, heated to 60 C for 30 hours and analyzed. Level of impurity (IV) was 30.2 area %

### Example 12

Same as in example 11, but without the addition of ZnCl₂. Level of impurity (IV) was 0.93 %.

### Example 13

Same as in example 12, but water had pH 10. Level of impurity (IV) was 0.92 %.

### Example 14

Same as in example 11, but water had pH 10. Level of impurity (IV) was 7.5%.

### Example 15

Same as in example 12, but water had pH 3. Level of impurity (IV) was 59.9 %.

### Example 16

1 g paliperidone (level of impurity (IV) was < 0,1 area %), 10 mL metanol, 0,3 mL water and in so much HCl(aq) or diisopropylamin, that the pH of suspension is according to the values written in the table were charged to the flask and heated to the reflux for 24 hours. Samples of suspensions were taken and analysed. Results are presented in the table below.

| Flask | pH suspension | Level of impurity (IV) / area % |
|---|---|---|
| 1 | 1.5 | 42.4 |
| 2 | 3.8 | 12.1 |
| 3 | 2.3 | 9.6 |
| 4 | 6.0 | 3.3 |
| 5 | Without addition of acid or base, 8.37 | 0.3 |
| 6 | 6.4 | 0.5 |
| 7 | 6.42 | 0.6 |
| 8 | 10.9 | 0.5 |
| 9 | 11.8 | 0.7 |

### Example 17

1 g of paliperidone (level of impurity (IV) was 0.75 area %), 30 mL of water and 1.86 g FeCl₂ (anhydrous) were charged in 50 mL three-necked flask, heated to 60 °C for 42 hours and analyzed. Levels of impurities (RRT 0.81 and RRT 1.29) were 60 and 14 area %

## Claims

1. A process for preparing paliperidone (I) by combining 3-(2-chloroethyl)-6,7,8,9-tetrahydro-4-hydroxy-2-methyl-4H-pyrrido[1,2-a]-pyrimidin-4-one (CMHTP) or a salt thereof and 6-fluom-3-piperidino-1,2-benzisoxazol (FBIP) or a salt thereof in an organic solvent and in the presence of water, wherein the pH value of the reaction medium is between 6 and 14, and wherein active charcoal is used in the crystalization step(s), having a neutral pH value and a content of individual metal cations less than 200 ppm.

2. A process according to claim 1, **characterized in that** the pH value of the reaction medium is between 8 and 10 and the metal cations are Zn²⁺ and Fe²⁺.

3. A process according to claims 1 or 2, **characterized in that** the reaction occurs in mixtures of an organic solvent, such as C1-8 alkyl alcohols, acetonitrile, C3-6 amides, C3-6 ketones, C5-12 aromatic hydrocarbons, C2-6 alkyl acetates and C2-8 ethers, with water.

4. A process according to any of claims 1 to 3, **characterized in that** the organic solvent is methanol.

5. A process according to any of claims 1 to 4, **characterized in that** the water fraction in the solvent mixture used is from 0.5 % (V/V) to 10% (V/V), preferably from 0.5% (V/V) to 5.0 % (V/V), most preferably from 0.5% (V/V) to 2.0 % (V/V).

6. A process according to any of claims to 3, **characterized in that** the reaction mixture is heated to a temperature of 50 °C to reflux, preferably to a temperature of 65°C to 70 °C.

7. A process according to any of claims 1 to 6, **characterized in that** during heating the reaction mixture such a temperature gradient is applied that a reaction mixture of 200 to 300 g is heated to the reaction temperature in more than 60 minutes, preferably more than 75 minutes.

8. A process according to any of claims 1 to 7, **characterized in that** the CMHTP used contains less than 0.5%, preferably less than 0.2% and more preferably less than 0.05% of impurity of the following formula (V)

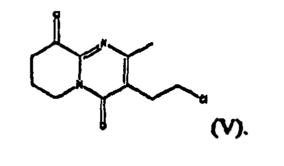

## Patentansprüche

1. Verfahren zur Herstellung von Paliperidon (I) durch Kombinieren von 3-(2-Chlorethyl)-6,7,8,9-tetrahydro-9-hydroxy-2-methyl-4H-pyrrido[1,2-a]-pyrimidin-4-on (CMHTP) oder einem Salz davon und 6-Fluor-3-piperidino-1,2-benzisoxazol (FBIP) oder einem Salz davon in einem organischen Lösungsmittel und in der Gegenwart von Wasser, wobei der pH-Wert des Reaktionsmediums zwischen 6 und 14 liegt, und wobei Aktivkohle in der bzw. den Kristallisationsstufe(n) verwendet wird, mit einem neutralen pH-Wert und einem Gehalt von individuellen Metallkationen von niedriger als 200 ppm.

2. Verfahren gemäß Anspruch 1, **dadurch gekennzeichnet, dass** der pH-Wert des Reaktionsmediums zwischen 8 und 10 liegt und die Metallkationen Zn²⁺ und Fe²⁺ sind.

3. Verfahren gemäß den Ansprüchen 1 oder 2, **dadurch gekennzeichnet, dass** die Umsetzung in Gemischen eines organischen Lösungsmittels, wie C1-8-AlkylAlkoholen, Acetonitril, C3-6-Amiden, C3-6-Ketonen, aromatischen C5-12-Kohlenwasserstoffen, C2-6-Alkyl-Acetaten und C2-8-Ethern, mit Wasser stattfindet.

4. Verfahren gemäß einem der Ansprüche 1 bis 3, **dadurch gekennzeichnet, dass** das organische Lösungsmittel Methanol ist.

5. Verfahren gemäß einem der Ansprüche 1 bis 4, **dadurch gekennzeichnet, dass** die Wasserfraktion in dem verwendeten Lösungsmittelgemisch 0,5 % (V/V) bis 10 % (V/V), bevorzugt 0,5 % (V/V) bis 5,0 % (V/V), am stärksten bevorzugt 0,5 % (V/V) bis 2,0 % (V/V) beträgt.

6. Verfahren gemäß einem der Ansprüche 1 bis 5, **dadurch gekennzeichnet, dass** das Reaktionsgemisch auf eine Temperatur von 50 °C bis Rückfluss, bevorzugt auf eine Temperatur von 65 °C bis 70 °C erwärmt wird.

7. Verfahren gemäß einem der Ansprüche 1 bis 6, **dadurch gekennzeichnet, dass** während dem Erwärmen des Reaktionsgemisches ein derartiger Temperaturgradient verwendet wird, dass ein Reaktionsgemisch von 200 bis 300 g auf die Reaktionstemperatur in mehr als 60 Minuten, bevorzugt mehr als 70 Minuten erwärmt wird.

8. Verfahren gemäß einem der Ansprüche 1 bis 7, **dadurch gekennzeichnet, dass** das verwendete CMHTP weniger als 0,5 %, bevorzugt weniger als 0,2 % und stärker bevorzugt weniger als 0,05 % Verunreinigung der folgenden Formel (V) enthält

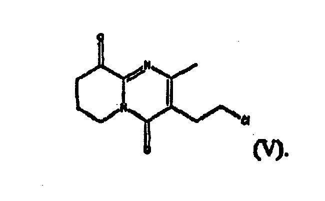

## Revendications

1. Procédé de préparation de la palipéridone (I) par combinaison de la 3-(2-chloroéthyl)-6,7,8,9-tétrahydro-9-hydroxy-2-méthyl-4H-pyrrido[1,2-a]pyrimidin-4-one (CMHTP) ou un sel de celle-ci et du 6-fluoro-3-pipéridino-1,2-benzisoxazole (FBIP) ou un sel de celui-ci, dans un solvant organique et en présence d'eau, où le pH du milieu réactionnel se situe dans l'intervalle allant de 6 à 14, et où du charbon actif est utilisé dans la ou les étapes de cristallisation, lequel a un pH neutre et une teneur en cations métalliques individuels inférieure à 200 ppm.

2. Procédé selon la revendication 1, **caractérisé en ce que** le pH du milieu réactionnel se situe dans l'intervalle allant de 8 à 10 et les cations métalliques sont Zn²⁺ et Fe²⁺.

3. Procédé selon la revendication 1 ou 2, **caractérisé en ce que** la réaction se produit dans des mélanges d'un solvant organique, tel que des alcools alkyliques en C1-8, l'acétonitrile, des amides en C3-6, des cétones en C3-6, des hydrocarbures aromatiques en C5-12, des acétates d'alkyle en C2-6 et des éthers en C2-8, avec de l'eau.

4. Procédé selon l'une quelconque des revendications 1 à 3, **caractérisé en ce que** le solvant organique est le méthanol.

5. Procédé selon l'une quelconque des revendications 1 à 4, **caractérisé en ce que** la fraction aqueuse dans le mélange de solvants utilisé se situe dans l'intervalle allant de 0,5% (v/v) à 10% (v/v), de préférence de 0,5% (v/v) à 5,0% (v/v), de manière la plus préférée de 0,5% (v/v) à 2,0% (v/v).

6. Procédé selon l'une quelconque des revendications 1 à 5, **caractérisé en ce que** le mélange réactionnel est chauffé jusqu'à une température allant de 50°C au reflux, de préférence à une température de 65°C à 70°C.

7. Procédé selon l'une quelconque des revendications 1 à 6, **caractérisé en ce que** pendant le chauffage du mélange réactionnel, on adopte un gradient de température tel qu'un mélange réactionnel de 200 à 300 g est chauffé à la température réactionnelle en plus de 60 minutes, de préférence plus de 75 minutes.

8. Procédé selon l'une quelconque des revendications 1 à 7, **caractérisé en ce que** la CMHTP utilisée contient moins de 0,5%, de préférence moins de 0,2% et de manière plus préférée, moins de 0,05% d'impureté de la formule (V) suivante :
